(19) Europäisches Patentamt
European Patent Office
Office européen des brevets

(11) Veröffentlichungsnummer: **0 052 870**
**A1**

# (12) EUROPÄISCHE PATENTANMELDUNG

(21) Anmeldenummer: 81109798.9

(22) Anmeldetag: 20.11.81

(51) Int. Cl.³: C 07 D 209/42
C 07 D 215/48, C 07 D 217/26
C 07 D 401/12, A 61 K 31/40
A 61 K 31/47

(30) Priorität: 25.11.80 DE 3044236

(43) Veröffentlichungstag der Anmeldung: 02.06.82 Patentblatt 82/22

(84) Benannte Vertragsstaaten: AT BE CH DE FR GB IT LI NL SE

(71) Anmelder: HOECHST AKTIENGESELLSCHAFT
Postfach 80 03 20
D-6230 Frankfurt/Main 80(DE)

(72) Erfinder: Geiger, Rolf, Prof. Dr.
Heinrich-Bleicher-Strasse 33
D-6000 Frankfurt am Main 50(DE)

(72) Erfinder: Teetz, Volker, Dr.
An der Tann 20
D-6238 Hofheim am Taunus(DE)

(72) Erfinder: Schölkens, Bernward, Dr.
Am Fliedergarten 1
D-6233 Kelkheim (Taunus)(DE)

## (54) Aminosäurederivate und Verfahren zu ihrer Herstellung.

(57) Gegenstand der Erfindung sind Verbindungen der Formel I

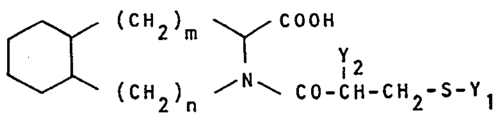

(I)

in welcher n 0 oder 1, m 1 oder 2, aber $(n+m) \leq 2$, $Y_1$ Wasserstoff, Niederalkanoyl oder -cycloalkanoyl, Benzoyl oder einen Rest der Formel II

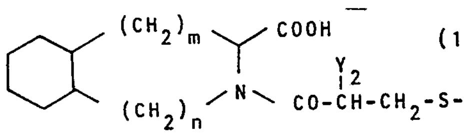

(II)

$Y_2$ Methyl, Amino, Niederalkanoylamido, Niedercycloalkanoylamido oder Benzoylamido bedeuten sowie deren Salze, deren Herstellung, daraus bestehende oder diese Verbindungen enthaltende Mittel und deren Verwendung zur Behandlung von Bluthochdruck.

EP 0 052 870 A1

Croydon Printing Company Ltd.

HOECHST AKTIENGESELLSCHAFT HOE 80/F 268 0052870
Dr.Li/Pa

## Aminosäurederivate und Verfahren zu ihrer Herstellung

Gegenstand der Erfindung sind Verbindungen der Formel

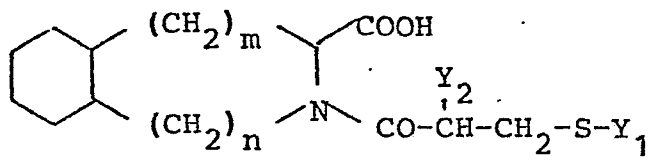

(I)

in welcher bedeuten: n 0 oder 1, m 1 oder 2, aber $(n+m) \leq 2$, $Y_1$ Wasserstoff, Niederalkanoyl oder -cycloalkanoyl, Benzoyl, oder einen Rest der Formel II, wobei vorzugsweise m und n jeweils in beiden Molekülhälften dieselbe Bedeutung haben,

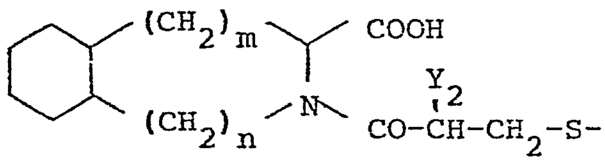

(II)

$Y_2$ Methyl, Amino, Niederalkanoylamido, Niedercycloalkanoylamido oder Benzoylamido.

Niederalkanoyl im Sinne des Vorstehenden bedeutet insbesondere Alkanoyl mit 1 bis 6 C-Atomen, Niedercycloalkanoyl insbesondere Cycloalkanoyl mit 5 bis 8 C-Atomen.

Gegenstand der Erfindung ist ferner ein Verfahren zur Herstellung dieser Verbindungen. Dieses ist dadurch gekennzeichnet, daß man einen Niederalkylester einer Iminosäure der Formel

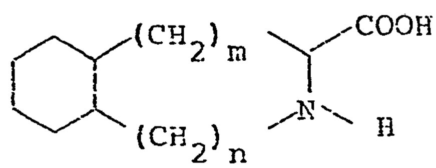

(III)

oder ein Salz dieser Verbindung mit einer starken, nicht flüchtigen Base mit einem aktivierten Derivat einer Carbonsäure der Formel IV

$$\begin{array}{c} Y_2 \\ | \\ HOOC\text{-}CH\text{-}CH_2\text{-}S\text{-}Y_1 \end{array} \qquad IV$$

in der $Y_1 \neq H$ ist, umsetzt, aus dem erhaltenen Produkt die Base bzw. die Estergruppe abspaltet, gegebenenfalls einen in der Position $Y_1$ stehenden Säurerest abspaltet oder durch einen anderen austauscht und, falls die Verbindung eine freie SH-Gruppe hat, gewünschtenfalls das Produkt zur Disulfidverbindung oxidiert.

Zur Synthese der erforderlichen Ausgangsverbindungen können die bekannten Verbindungen Indol-2-carbonsäure, Chinolin-2-carbonsäure oder 1.2.3.4-Tetrahydroisochinolin-3-carbonsäure katalytisch hydriert werden. Bevorzugte Katalysatoren sind Platin und Rhodium, die bei Temperaturen bis etwa 100°C und Drucken bis etwa 150 bar verwendet werden.
Die Carbonsäuren der Formel IVa

$$\begin{array}{c} Y^1\text{-}S\text{-}CH_2\text{-}CH\text{-}COOH \\ | \\ CH_3 \end{array} \qquad (IVa)$$

sind unter anderem in der DE-OS 2 752 720 beschrieben oder können analog hergestellt werden.
Geeignete Derivate des Cysteins sind literaturbekannt.

Die Herstellung der Säureamidbindung folgt den allgemeinen Verfahren, bevorzugt denen der Peptidchemie, wie sie beispielsweise in Houben-Weyl, Methoden der organischen Chemie, Band 15, ausführlich beschrieben sind.

Als aktivierte Derivate einer Carbonsäure der Formel IV kommen z.B. die Säurechloride, Aktivester, gemischte Anhydride mit beliebigen anderen Carbonsäuren oder Kohlensäurehalbestern in Frage. Sie lassen sich mit Salzen der

Iminosäuren der Formel III direkt umsetzen. Unter Basen werden in erster Linie Alkali- oder Erdalkalihydroxyde verstanden. Daneben kommen als starke, nicht flüchtige organische Basen im Sinne der Erfindung beispielsweise Tetraalkyl- oder-aralkylammoniumhydroxyde oder tetraalkylierte Guanidine wie Tetramethylguanidin infrage.

Aktivierte Ester können z.B. aus der entsprechenden Säure und N-Hydroxysuccinimid, 2,4,5-Trichlorphenol oder 1-Hydroxybenzotriazol in bekannter Weise mit Dicyclohexylcarbodiimid hergestellt und nach dem Abtrennen des bei der Herstellung entstehenden Dicyclohexylharnstoffs in Lösung sogleich verwendet werden. Bevorzugte Lösungsmittel sind Dimethylformamid, Dimethylacetamid, N-Methylpyrrolidon, Phosphorsäure-tris-dimethylamid oder Methylenchlorid. In denselben Lösungsmitteln werden die erfindungsgemäßen Salze der Iminosäuren der Formel III aufgenommen, die zuvor, z.B. in alkoholischer oder wäßrig-alkoholischer Lösung, aus den Komponenten in äquimolarer Menge erhalten und vom Lösungmittel, z.B. durch Abdestillieren im Vakuum, befreit wurden.

Verbindungen, in denen $Y_1$ Wasserstoff ist, können durch Behandeln der entsprechenden S-Acylderivate mit Ammoniak oder Alkali- bzw. Erdalkalihydroxiden erhalten werden.

Die Reaktion kann bei Raumtemperatur stattfinden und ist bei Verwendung der Säurechloride nach etwa 30 Minuten, bei Verwendung von Aktivestern nach längstens 14 Stunden beendet. Man trennt dann das Lösungsmittel, z.B. durch Destillieren im Vakuum, ab und reinigt das Rohprodukt z.B. durch Flüssigkeitschromatographie an Silicagel, wobei sich Lösungsmittelsysteme mit Chloroform, Methanol und Essigsäure bewährten.

Mann kann aber auch, wie das bevorzugt in der Peptidchemie geschieht, die Carbonsäuren IV mit Alkylestern der Iminosäuren der Formel III kondensierten und die Ester anschließend spalten oder verseifen, wobei durch Alkali gleichzeitig die S-Acylbindung gespalten wird. Bevorzugtes Kondensationsmittel ist Dicyclohexyl-carbodiimid, gegebenenfalls unter Zusatz von N-Hydroxysuccinimid oder 1-Hydroxybenzotriazol. Bevorzugte Lösungsmittel sind die oben angeführten.

So kann man beispielsweise auch einen Ester der Formel V

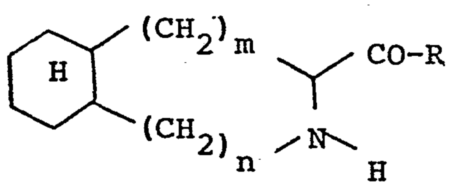

(V)

in der R den Rest eines sauer abspaltbaren Alkohols, z.B. tert.-Butoxy darstellt, mit einer Carbonsäure der Formel IV umsetzten. In den so erhaltenen Verbindungen kann man nach saurer Spaltung des Esters den Rest $Y^1$ durch Wasserstoff ersetzen, indem man das Produkt mit Ammoniak, einem primären oder sekundären Amin oder einem Alkali- oder Erdalkalihydroxid behandelt. Durch Weiterbehandeln mit Säure oder einem stark sauren Ionenaustauscher wird die Verbindung der Formel I mit $Y^1$ = H freigesetzt.

Man kann auch andere Alkylester, bevorzugt Methyl- oder Ethylester, der Dekahydro-isochinolin-3-carbonsäure mit einer Carbonsäure der Formel IV kondensieren und das Reaktionsprodukt zur Abspaltung der Ester- und der S-Acylgruppe mit einem Alkali- oder Erdalkalihydroxid behandeln.

Verbindungen der Formel I mit $Y_1$ = H und $Y_2$ = Amino können dadurch hergestellt werden, daß man eine Verbindung der Formel V mit N,S-Di-tert.-butyloxycarbonylcystein kondensiert und die Schutzgruppen durch Behandeln mit einer starken Säure, bevorzugt in Anwesenheit eines SH-Gruppen tragenden Kationenfängers wie Thiophenol oder Ethylmercaptan,

abspaltet.

In die erhaltenen Produkte mit $Y^1$ = H können durch Behandeln mit einem milden Oxydationsmittel wie Luft, Jod oder Kaliumhexacyanoferrat-(III) Reste der Formel II für $Y^1$ eingeführt werden.

Zur Herstellung von Verbindungen der Formel I, in der $Y^1$ einen Rest der Formel II darstellt, kann man beispielsweise auch eine Verbindung der Formel III, ihre Salze oder Ester mit einer Verbindung der Formel VI

$$HOOC-\overset{Y^2}{\overset{|}{C}H}-CH_2-S-S-CH_2-\overset{Y^2}{\overset{|}{C}H}-COOH \qquad (VI)$$

umsetzen und evtl. vorhandenen Schutzgruppen gewünschtenfalls abspalten.

Je nach Charakter der Ausgangsstoffe, insbesondere der Bedeutung der Substituenten $Y^1$ und $Y^2$, kann das eine oder andere der beschriebenen Verfahren in einzelnen Fällen eine gewünschte individuelle Verbindung nur in geringen Ausbeuten liefern oder zu deren Synthese nicht geeignet sein. In solchen verhältnismäßig selten auftretenden Fällen macht es dem Fachmann keine Schwierigkeiten, das gewünschte Produkt auf einem anderen der beschriebenen Verfahrenswege zu synthetisieren.

Die Indentität der erfindungsgemäßen Verbindungen wird durch Elementaranalyse und NMR-Spektroskopie gesichert.

Die erfindungsgemäßen Verbindungen erniedrigen bei peroraler Gabe von 0,5 bis 20 mg pro kg und Tag an der hypertonen Ratte und am Hund den Blutdruck und sind auch am Menschen in demselben Dosisbereich wirksam. Sie sind 2-4 mal wirksamer als die entsprechenden benzoiden Verbindungen mit Indolin-2-carbonsäure, 1,2,3,4-Tetrahydrochinolin-2-carbonsäure oder 1,2,3,4-Tetrahydroisochinolin-3-carbonsäure und

solche, bei denen die Carbonsäure der Formel IV säureamidartig mit Prolin verbunden ist. Die Dosis konnte im Tierversuch ohne schädliche Wirkung erhöht werden.

Die neuen Verbindungen werden zur Bekämpfung des Bluthochdrucks verschiedener Genese eingesetzt. Sie können für sich oder in Kombination mit anderen blutdrucksenkenden, gefäßerweiternden oder diuretisch wirksamen Verbindungen angewandt werden. Typische Vertreter dieser Wirkklasse sind z.B. in Erhardt-Ruschig, Arzneimittel, 2.Auflage, Weinheim 1972, beschrieben.

Die Erfindung wird durch folgende Beispiele weiter erläutert:

Beispiel 1

N-(2-Methyl-3-acetylmercaptopropionyl)-L-dekahydro-isochinolin-3-carbonsäure

A. N-Benzyloxycarbonyl-L-dekahydro-isochinolin-3-carbonsäure

36 g L-Dekahydro-isochinolin-3-carbonsäure, hergestellt aus der 1,2,3,4-Tetrahydroverbindung durch katalytische Hydrierung an 5 % Rhodium auf Kohle bei 60 - 90°C und 80 - 150 bar $H_2$-Druck in 90 % Essigsäure, werden in 200 ml 1N NaOH suspendiert. Unter starkem Rühren gibt man bei 0-5°C 30.2 ml Benzyloxycarbonylchlorid und 232 ml 1 N NaOH gleichzeitig langsam zu und rührt noch 2 Stunden. Dann wird ungeachtet eines Niederschlags zweimal mit Ether extrahiert, die wäßrige Lösung mit konzentrierter Salzsäure angesäuert und mit Essigester ausgeschüttelt. Die Essigesterphase wird mit Wasser gewaschen und über $Na_2SO_4$ getrocknet. Nach Abdestillieren des Lösungsmittels im Vakuum hinterbleibt ein Harz. Es wird in heißem Diisopropylether gelöst. Nach Versetzen mit Dicyclohexylamin fällt beim Abkühlen das Salz aus. Ausbeute 49,1 g. Freisetzen der Säure mit Kaliumhydrogensulfat/Essigester in üblicher Weise.

B. N-Benzyloxycarbonyl-L-dekahydro-isochinolin-3-carbonsäure-tert.-butylester

32 g der nach A erhaltenen Verbindung werden in 250 ml Methylenchlorid gelöst. Man gibt 38.5 ml tert.-Butanol und 1 g 4-Dimethylaminopyridin zu und tropft unter Rühren bei 0°C die Lösung von 22 g Dicyclohexylcarbodiimid in 60 ml Methylenchlorid zu. Man rührt dann 15 Minuten bei 0°C und 5 h bei Raumtemperatur, filtriert und bringt das Filtrat im Vakuum zur Trockene. Der Rückstand wird wieder in Methylenchlorid aufgenommen, die Lösung nacheinander mit gesättigter Natriumhydrogencarbonatlösung und Kaliumhydrogensulfat-/Kaliumsulfatlösung ausgeschüttelt, mit Wasser neutral gewaschen und über $Na_2SO_4$ getrocknet.

Nach dem Eindampfen hinterbleibt ein öliger Rückstand. Ausbeute 32,0 g.

C. L-Dekahydro-isochinolin-3-carbonsäure-tert.-butylester-toluolsulfonat

30,5 g der nach B erhaltenen Verbindung werden in 300 ml Methanol gelöst und an Palladiummohr katalytisch hydriert, wobei am Autotitrator durch Zugabe von 1N Toluolsulfosäure in Methanol der pH-Wert bei 5 gehalten wird. Nach beendeter Reaktion wird der Katalysator abfiltriert und das Lösungsmittel abdestilliert. Der harzige Rückstand wird beim Verreiben mit Ether und Wasser fest. Ausbeute 31,4 g. Die Verbindung ist chromatographisch nahezu rein und vom Ausgangsmaterial durch einen niedrigeren $R_f$-Wert im DC (Kieselgel) unterschieden.

D. N-(2-Methyl-3-acetylmercaptopropionyl)-L-dekahydro-isochinolin-carbonsäure-tert.-butylester

Man löst 20,5 g des nach C erhaltenen tert.-Butylester-tosylats in 200 ml Methylenchlorid, gibt 8,3 g 2-Methyl-3-acetylmercapto-propionsäure, 6,4 ml N-Ethylmorpholin und unter Eiskühlung 11 g Dicyclohexylcarbodiimid, in 60 ml Methylenchlorid gelöst, zu. Man rührt über Nacht bei Raumtemperatur, filtriert, destilliert das Lösungsmittel im Vakuum ab und nimmt den Rückstand in Essigester auf. Die Lösung wird nacheinander mit $NaHCO_3$-Lösung, $KHSO_4/K_2SO_4$-Lösung und Wasser gewaschen, über $Na_2SO_4$ getrocknet und im Vakuum eingedampft.
Man erhält 15,9 g Öl.
NMR ($CDCl_3$): 1,20 (d, 3H); 1,40 (s, 9H); 2,13 (s, 3H); 1,1 - 5,1 (m, 18H).

E. N-(2-Methyl-3-acetylmercaptopropionyl)-L-dekahydro-isochinolin-3-carbonsäure

13 g der nach D erhaltenen Verbindung löst man in 70 ml Trifluoressigsäure, die nach 45 min. im Vakuum abdestilliert wird. Man verteilt den Rückstand zwischen

180 ml Ethylether und 90 ml gesättigter $NaHCO_3$-Lösung. Die Etherphase wird verworfen, die wäßrige Phase mit konzentrierter Salzsäure angesäuert und dreimal mit Essigester extrahiert. Die vereinigten Essigesterlösungen werden mit Wasser gewaschen und über $Na_2SO_4$ getrocknet. Man destilliert das Lösungsmittel ab, nimmt den Rückstand in Ether auf und versetzt unter Rühren solange mit Cyclohexylamin, bis feuchtes pH-Papier einen Wert von 8 anzeigt. Nach Kühlen der entstandenen Suspension filtriert man das Cyclohexylammoniumsalz ab und wüscht es mit Ether. Nach dem Trocknen wird es aus Isopropanol und Acetonitril umkristallisiert. Ausbeute 4,6 g. Zur Gewinnung der freien Säure wird das Salz zwischen Wasser und Essigester/Ether suspendiert. Nach Zugabe von $KHSO_4$-Lösung bis pH 2 geht alles in Lösung. Man trennt die Phasen, wäscht die wäßrige Lösung mit etwas Essigester und die vereinigten, organischen Phasen mit wenig Wasser, trocknet diese über $Na_2SO_4$ und destilliert das Lösungsmittel im Vakuum ab. Der harzige Rückstand wird beim Verreiben mit Ether fest. Ausbeute 3,0 g. Elementaranalyse korrekt.
NMR (DMSO-$d_6$): 1,22 (d, 3H); 2,15 (s, 3H)
1,1 - 5,1 (m, 18 H).

Beispiel 2

N-(2-Methyl-3-mercaptopropionyl)-L-dekahydro-isochinolin-3-carbonsäure

1,5 g der nach Beispiel 1 E hergestellten Verbindung werden in 8 ml 5N Ammoniak in Methanol 2 Stunden unter Stickstoff aufbewahrt. Dann bringt man die Lösung im Vakuum zur Trockne. Der feste Rückstand wird in 10 ml 50 %igem, wäßrigem Methanol gelöst. Man filtriert über eine kleine Säule mit dem stark sauren Ionenaustauscher Lewatit S 100 und bringt das Eluat im Vakuum zur Trockne. Der harzige Rückstand wird mit Petrolether digeriert, wobei er fest wird. Nach Trocknen im Vakuum erhält man die Titelverbindung in einer Ausbeute von 0,81 g. Elementaranalyse korrekt.

NMR ($CF_3COOH$): 1,20 (d, 3H); 1,2 - 5,2 (m, 19H).

Beispiel 3

N-D-Cysteinyl-L- dekahydro-isochinolin-3-carbonsäure

A. N.S.-Di-Boc-D-cysteinyl-L-dekahydro-isochinolin-3-carbonsäure-tert.-butylester

4,36 g N,S-Di-Boc-D-cystein-N-hydroxysuccinimidester, hergestellt analog Liebig's Ann. Chem. 743 (1971), Seite 57, und 4,1 g L-Dekahydro-isochinolin-3-carbonsäure-tert.-butylester-tosylat, hergestellt nach Beispiel 1, werden in 60 ml Dimethylformamid gelöst. Man gibt 1,28 ml N-Ethylmorpholin zu und rührt über Nacht bei Raumtemperatur. Dann destilliert man das Lösungsmittel im Vakuum ab, digeriert den harzigen Rückstand mit Wasser und trocknet im Vakuum. Ausbeute 4,38 g einer festen Masse von unscharfem Schmelzpunkt.

B. D-Cysteinyl-L-dekahydro-isochinolin-3-carbonsäure

4,4 g der nach A erhaltenen Verbindung werden 15 - 20 Minuten in 0,2N HCl in Ameisensäure, die 10 % Ethylmercaptan enthält, bei Raumtemperatur aufbewahrt. Man bringt im Vakuum zur Trockne und digeriert den Rückstand mit Ether. Das erhaltene feste Produkt ist etwas hygroskopisch. Ausbeute 1.44 g (56 %). Elementaranalyse nach Berücksichtigung von 3-5 % Wasser korrekt.

Beispiel 4

N-Acetyl-S-Benzoyl-D-cysteinyl-L-dekahydro-isochinolin-3-carbonsäure

Man setzt 3,9 g N-Acetyl-S-benzoyl-D-cystein-4-nitrophenylester, erhalten analog J. Org. Chem. 27 (1962), Seite 3329, nach Beispiel 3 A mit 4,2 g L-Dekahydro-isochinolin-3-carbonsäure-tert.-butylester-tosylat und 1,28 ml N-Ethylmorpholin um. Das Rohprodukt wird durch Filtration in chloroformischer Lösung über Silicagel gereinigt. Ausbeute 3,52 g einer halbfesten Masse, die in der DC einheitlich ist und eine befriedigende Elementaranalyse zeigt.

Zur Abspaltung des tert.-Butylalkohols aus der Estergruppe bewahrt man die Verbindung 40 Minuten in Trifluoressigsäure auf, die sodann im Vakuum abdestilliert wird. Der Rückstand wird in 50 %igem, wäßrigem Methanol gelöst und mit einem schwach basischen Ionenaustauscher in der Acetatform behandelt, bis der pH-Wert von etwa 2 auf etwa 3-4 zurückgegangen ist. Dann wird vom Austauscher abfiltriert. Nach Abdestillieren des Lösungsmittels im Vakuum und Trocknen über $P_2O_5$ erhält man eine harte, pulverisierbare Masse. Ausbeute 2,1 g. Zur Analyse wird das Cyclohexylammonium- oder Dicyclohexylammoniumsalz aus Isopropanol umkristallisiert. Elementaranalyse der Salze korrekt.

Beispiel 5

N-Acetyl-D-cysteinyl-L-dekahydro-isochinolin-3-carbonsäure

1 g der nach Beispiel 4 erhaltenen Verbindung wird in 4N methanolischem Ammoniak gelöst und 2 Stunden unter Stickstoff aufbewahrt. Aufarbeitung nach Beispiel 2. Ausbeute 0,50 g.
NMR ($CF_3COOH$): 2,1 (s, 3H); 1,1-5,2 (20H).

Beispiel 6

(2-Methyl-3-acetylmercaptopropionyl-L-dekahydro-isochinolin-3-carbonsäure

A. 2-Methyl-3-acetylmercaptopropionsäure-2,4,5-trichlorphenylester

Man stellt in bekannter Weise aus 16,2 g der Säure, 19,7 g 2,4,5-Trichlorphenol und 22 g Dicyclohexylcarbodiimid in Tetrahydrofuran den Trichlorphenylester her. Nach Filtration wird die Verbindung durch Chromatographie an Silicagel mit Tetrahydrofuran als Eluans gereinigt. Ausbeute 29,8 g (79 %). Schmp. 40-41°.

B. (2-Methyl-3-acetylmercaptopropionyl)-L-dekahydro-isochinolin-3-carbonsäure

Man stellt aus 1,8 g Dekahydro-isochinolin-3-carbonsäure und 1,05 g Tetramethylguanidin in 10 ml Methanol unter Stickstoff das entsprechende Salz her, destilliert das

Methanol im Vakuum ab und löst den Rückstand unter Stickstoff in 10 ml Dimethylformamid. Zu dieser Lösung gibt man 3,4 g des nach A erhaltenen 2-Methyl-3-acetylmercaptopropionsäure-aktivesters und 0,1 g 1-Hydroxybenzotriazol in 10 ml Dimethylformamid und bewahrt das ganze über Nacht bei Raumtemperatur auf. Nach Abdestillieren des Lösungsmittels reinigt man wie in Beispiel 1E beschrieben und erhält 0,95 g der Titelverbindung, die mit der nach Beispiel 1E erhaltenen Verbindung identisch ist.

Beispiel 7

N-(2-Methyl-3-mercaptopropionyl)-L-dekahydro-isochinolin-3-carbonsäure

17,9 g Dekahydro-isochinolin-3-carbonsäure werden in bekannter Weise durch Salzsäure oder Thionylchlorid in Methanol in das Methylester-hydrochlorid überführt. Die Verbindung wird nach Abdestillieren des Lösungsmittels durch Digerieren mit Ether isoliert.

Den trockenen Rückstand setzt man nach Beispiel 1 D mit 16,2 g 2-Methyl-3-acetylmercaptopropionsäure, 12,8 ml N-Ethylmorpholin und 22 g Dicyclohexyl-carbodiimid in Methylenchlorid um. Aufarbeitung analog Beispiel 1 D.

Der ölige Rückstand wird in 150 ml Dioxan-Methanol 1:1 gelöst. Man gibt unter Rühren und unter Stickstoff 2N NaOH tropfenweise zu und hält den pH-Wert bei 12,5 - 13. Wenn keine Natronlauge mehr verbraucht wird, behandelt man die Lösung mit stark saurem Ionenaustauscher in 50 %igem, wäßrigen Methanol und engt die vereinigten Lösungen im Vakuum ein. Der Rückstand wird mit Petrolether degeriert und im Vakuum getrocknet. Ausbeute 7,8 g. Die Verbindung ist in der Dünnschichtchromatographie mit der nach Beispiel 2 erhaltenen identisch.

Beispiel 8

ß,ß-Dithio-diisobutyryl-bis-L-dekahydro-isochinolin-3-carbonsäure

A. 4,2 g L-Dekahydro-isochinolin-3-carbonsäure-tert.-butylester-tosylat werden mit 1,2 g ß,ß-Dithiodiisobuttersäure, hergestellt nach Svensk kem. Tidskr. 55 (1943) Seite 170, und 1,1 g Dicyclohexylcarbodiimid in 50 ml Dimethylformamid in Anwesenheit von 1,28 nl N-Ethylmorpholin umgesetzt. Das Reaktionsprodukt wird nach Filtration mit Wasser ausgefällt und getrocknet. Zur Spaltung des tert.-Butylesters behandelt man das Produkt 45 Minuten mit Trifluoressigsäure, die alsdann im Vakuum abdestilliert wird. Der Rückstand wird in 80 ml Methanol gelöst, die Lösung mit wenig schwach basischem Ionenaustauscher in der Acetatform verrührt und nach Filtration im Vakuum zur Trockene gebracht. Es entsteht eine harzige Masse, die nach längerem Stehen unter Ether/Petrolether fest wird und sich verreiben läßt. Sie wird vorteilhaft in ein Alkali- oder Ammoniumsalz überführt und ist dann besser zu handhaben.

B. 2 g der nach Beispiel 2 hergestellten Verbindung werden in einem Gemisch aus Ethanol und Natriumphosphatpuffer pH 6 gelöst. Man gibt unter Rühren tropfenweise Jod bis zur gleichbleibenden Gelbfärbung zu, entfärbt mit Thiosulfat und destilliert das Ethanol im Vakuum ab. Nun wird mit 4N Salzsäure vorsichtig bis pH 1.5 - 2 angesäuert und die Titelverbindung mit Essigester ausgeschüttelt. Die Essigesterlösung wird mit wenig Wasser gewaschen, über $Na_2SO_4$ getrocknet und im Vakuum eingedampft. Ausbeute 1,77 g. Zur Analyse wird in Acetonitril das Cyclohexylaminsalz hergestellt. Elementaranalyse korrekt.

Beispiel 9

3-Mercapto-2-methylpropanoyl-dekahydrochinolin-2-carbonsäure

1,63 g 2-Methyl-3-acetylmercapto-propionsäure und 2,4 g

Dekahydrochinolin-2-carbonsäuremethylesterhydrochlorid, werden unter Stickstoff in 20 ml Dimethylacetamid gelöst. Man gibt ferner nacheinander 1,35 g 1-Hydroxy-benzotriazol, 1,28 ml N-Ethylmorpholin und 2,2 g Dicyclohexylcarbodiimid zu und rührt 4 Stunden bei Raumtemperatur. Dann filtriert man und destilliert aus dem Filtrat das Lösungsmittel im Vakuum ab. Der Rückstand wird in 30 ml Methanol gelöst. Man rührt und tropft unter Stickstoff 10 ml 4N NaOH zu und versetzt 10 Minuten nach beendeter Zugabe mit 10 ml 4N HCl. Dann wird das Gemisch im Vakuum zur Trockene gebracht. Nach Abdestillieren des Lösungsmittels reinigt man durch Chromatographie an 130 g $SiO_2$ im System Chloroform/Isopropanol/Essigsäure (50:10:3). Ausbeute 2,2 g. Elementaranalyse korrekt.

NMR ($CF_3COOH$): 1,21 (d, 3H); 1,2-3,8 (m, 12H); 4,96 (m, 1H).

Beispiel 10

2-Methyl-3-acetylmercaptopropionyl-oktahydroindol-2-carbonsäure

Man stellt aus 2-Methyl-3-acetylmercaptopropionsäure mit 10 % Überschuß Thionylchlorid in Methylenchlorid durch dreistündiges Erwärmen das Säurechlorid her, das durch Destillation bei 1 mm Hg rein erhalten wird.

1,9 g des Säurechlorids werden mit dem Tetramethylenguanidiniumsalz von 1,63 g Indolin-2-carbonsäure in Dimethylacetamid unter Stickstoff umgesetzt. Man erhält nach Aufarbeitung und Reinigung analog Beispiel 10 2,9 g reine Titelverbindung mit korrekter Elementaranalyse.

Beispiel 11

2-Methyl-3-mercaptopropionyl-oktahydroindol-2-carbonsäure

Man spaltet von der nach Beispiel 10 hergestellten Verbindung analog Beispiel 2 die Acetylgruppe ab und arbeitet wie dort beschrieben auf. Harz, Elementaranalyse korrekt.

Patentansprüche:

1. Verbindung der Formel I

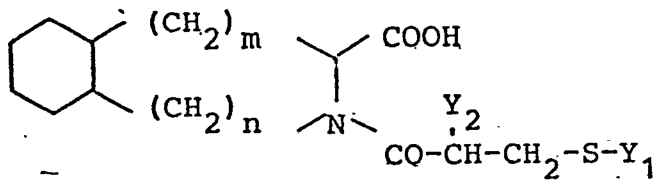

(I)

in welcher bedeuten:

n 0 oder 1,

m 1 oder 2, aber $(n + m) \leq 2$

$Y_1$ Wasserstoff, Niederalkanoyl oder -cycloalkanoyl, Benzoyl oder einen Rest der Formel II

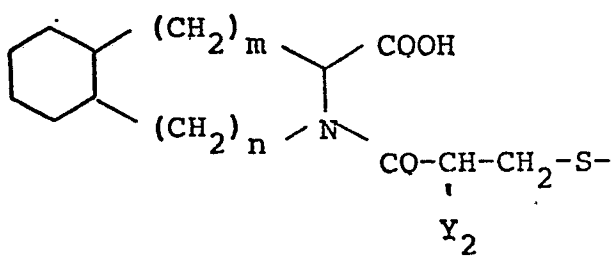

(II)

$Y_2$ Methyl, Amino, Niederalkanoylamido, Niedercycloalkanoylamido oder Benzoylamido

sowie deren Salze.

2. Verbindung gemäß Anspruch 1, in der m=n=1, $Y_1$ Wasserstoff und $Y_2$ Methyl ist.

3. Verbindung gemäß Anspruch 1, in der m=n=1, $Y_1$ Acetyl und $Y_2$ Methyl ist.

4. Verbindung gemäß Anspruch 1, in der m=n=1, $Y_1$ aus Wasserstoff und $Y_2$ Amino ist.

5. Verbindung gemäß Anspruch 1, in der m=1, n=0, $Y_1$ Acetyl, Benzoyl oder Wasserstoff und $Y_2$ Methyl ist.

6. Verbindung gemäß Anspruch 1, in der m=1, n=0, $Y_1$ Wasserstoff und $Y_2$ Amino ist.

7. Verfahren zur Herstellung einer Verbindung der Formel I, dadurch gekennzeichnet, daß man

einen Niederalkylester einer Verbindung der Formel III

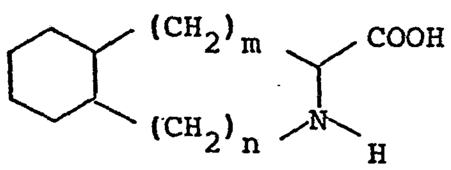

(III)

oder ein Salz dieser Verbindung mit einer starken, nicht flüchtigen Base mit einem aktivierten Derivat einer Carbonsäure der Formel IV

$$HOOC-\overset{Y_2}{\overset{|}{CH}}-CH_2-S-Y_1 \qquad (IV)$$

in der $Y_1 \neq H$ ist, umsetzt und aus dem erhaltenen Produkt die Base bzw. die Estergruppe abspaltet oder

einen Ester der Oktahydroindolin-2-,Dekahydro-chinolin-2- oder -isochinolin-3-carbonsäure der Formel V

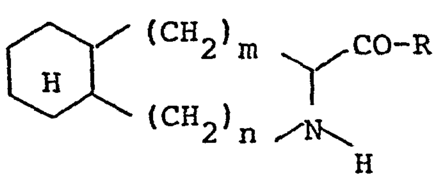

(V)

in der R den Rest eines sauer abspaltbaren Alkohols darstellt, mit einer Carbonsäure der Formel IV kondensiert und das Reaktionsprodukt zur Abspaltung der Ester- und der S-Acylgruppe zunächst mit einer starken Säure und dann mit Ammoniak oder einem Alkali- oder Erdalkalihydroxid behandelt oder

falls $Y_1$ = H und $Y_2$ = Amino ist, eine Verbindung der Formel V mit N,S-Di-tert. -butyloxycarbonylcystein kondensiert und die Schutzgruppen durch Behandeln mit einer starken Säure abspaltet oder

ein Tetraalkylguanidinium-, Trialkyl-benzyl- oder Tetraalkylammoniumsalz der Oktahydroindolin-2-,Dekahydro-chinolin-2- oder -isochinolin-3-carbonsäure mit einem aktivierten Derivat einer Carbonsäure der Formel IV umsetzt und das erhaltene Salz mit Säure oder einem stark sauren Ionenaustauscher zerlegt oder,

falls $Y^1$ einen Rest der Formel II darstellt, die Oktahydroindolin-2-, Dekahydro-chinolin-2- oder -isochinolin-3-carbonsäure, ihre Salze oder Ester mit Verbindungen der Formel VI

$$HOOC - \underset{\substack{|\\Y_2}}{CH} - CH_2 - S - S - CH_2 - \underset{\substack{|\\Y_2}}{CH} - COOH \qquad VI$$

kondensiert und gewünschtenfalls evtl. vorhandene Schutzgruppen abspaltet oder

gegebenenfalls geschützte Verbindungen der Formel I mit Acylgruppen in der Position $Y^1$, durch Behandeln mit Ammoniak, einem Alkali- oder Erdalkalihydroxyd und gegebenenfalls Behandeln mit einem milden Oxidationsmittel weiter modifiziert.

8. Verfahren nach Anspruch 7, dadurch gekennzeichnet, daß die Base mittels einer Säure oder eines stark sauren Ionenaustauschers abgespalten wird.

9. Verfahren nach Anspruch 7, dadurch gekennzeichnet, daß der in der Position $Y_1$ stehende Säurerest mit Alkali abgespalten wird.

10. Mittel, bestehend aus oder enthaltend eine Verbindung gemäß Anspruch 1.

11. Verwendung der Verbindungen gemäß Anspruch 1 zur Behandlung des Bluthochdrucks.

12. Verwendung der Verbindungen gemäß Anspruch 1 in Kombination mit einem Diuretikum zur Behandlung des Bluthochdrucks.

Patentansprüche (Österreich):

1. Verfahren zur Herstellung einer Verbindung der Formel I,

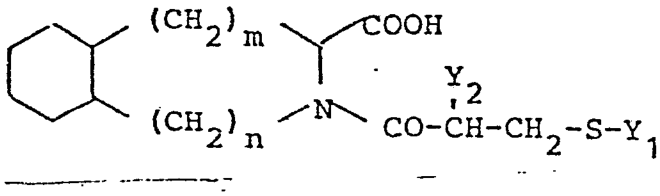

(I)

in welcher bedeuten:

n 0 oder 1,

m 1 oder 2, aber $(n + m) \leq 2$

$Y_1$ Wasserstoff, Niederalkanoyl oder -cycloalkanoyl, Benzoyl oder einen Rest der Formel II

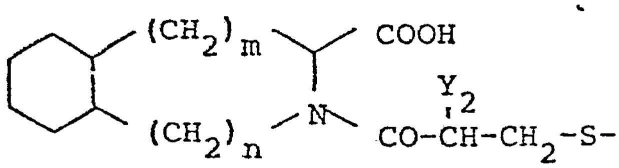

(II)

$Y_2$ Methyl, Amino, Niederalkanoylamido, Niedercycloalkanoylamido oder Benzoylamido,

sowie deren Salze, dadurch gekennzeichnet, daß man einen Niederalkylester einer Verbindung der Formel III

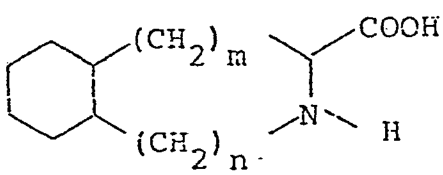

(III)

oder ein Salz dieser Verbindung mit einer starken, nicht flüchtigen Base mit einem aktivierten Derivat einer Carbonsäure der Formel IV

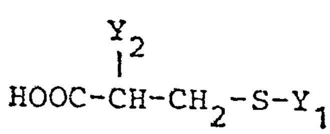

IV

in der $Y_1 \neq H$ ist, umsetzt und aus dem erhaltenen Produkt die Base bzw. die Estergruppe abspaltet oder

einen Ester der Oktahydroindolin-2-, Dekahydro-chinolin-2- oder -isochinolin-3-carbonsäure der Formel V

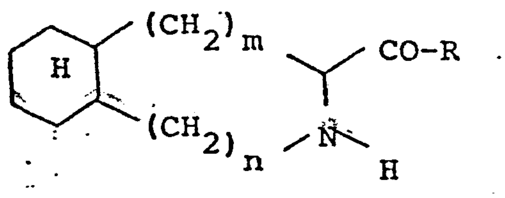

(V)

in der R den Rest eines sauer abspaltbaren Alkohols darstellt, mit einer Carbonsäure der Formel IV kondensiert und das Reaktionsprodukt zur Abspaltung der Ester- und der S-Acylgruppe zunächst mit einer starken Säure und dann mit Ammoniak oder einem Alkali- oder Erdalkalihydroxid behandelt oder
falls $Y_1$ = H und $Y_2$ = Amino ist, eine Verbindung der Formel V mit N,S-Di-tert.-butyloxycarbonylcystein kondensiert und die Schutzgruppe durch Behandeln mit einer starken Säure abspaltet oder
ein Tetraalkylguanidinium-, Trialkyl-benzyl- oder Tetraalkylammoniumsalz der Oktahydroindolin-2-, Dekahydro-chinolin-2- oder isochinolin-3-carbonsäure mit einem aktivierten Derivat einer Carbonsäure der Formel IV umsetzt und das erhaltene Salz mit Säure oder einem stark sauren Ionenaustauscher zerlegt oder,
falls $Y_1$ einen Rest der Formel II darstellt, die Oktahydroindolin-2-, Dekahydro-chinolin-2- oder -isochinolin-3-carbonsäure, ihre Salze oder Ester mit Verbindungen der Formel VI

$$\begin{array}{c} \quad Y_2 \qquad\qquad\quad Y_2 \\ HOOC-CH-CH_2-S-S-CH_2-CH-COOH \end{array} \qquad (VI)$$

kondensiert und gewünschtenfalls evt. vorhandene Schutzgruppen abspaltet oder
gegebenenfalls geschützte Verbindungen der Formel I mit Acylgruppen in der Position $Y_1$ durch Behandeln mit Ammoniak, einem Alkali- oder Erdalkalihydroxid und gegebenenfalls Behandeln mit einem milden Oxidationsmittel weiter modifiziert und die erhaltenen Verbindungen gegebenenfalls in die Salze überführt.

2. Verfahren gemäß Anspruch 1, dadurch gekennzeichnet, daß $m = n = 1$, $Y_1$ Wasserstoff und $Y_2$ Methyl ist.

3. Verfahren gemäß Anspruch 1, dadurch gekennzeichnet, daß $m = n = 1$, $Y_1$ Acetyl und $Y_2$ Methyl ist.

4. Verfahren gemäß Anspruch 1, dadurch gekennzeichnet, daß $m = n = 1$, $Y_1$ aus Wasserstoff und $Y_2$ Amino ist.

5. Verfahren gemäß Anspruch 1, dadurch gekennzeichnet, daß $m = 1$, $n = 0$, $Y_1$ Acetyl, Benzoyl oder Wasserstoff und $Y_2$ Methyl ist.

6. Verfahren gemäß Anspruch 1, dadurch gekennzeichnet, daß $m = 1$, $n = 0$, $Y_1$ Wasserstoff und $Y_2$ Amino ist.

7. Verfahren nach Anspruch 1 bis 6, dadurch gekennzeichnet, daß die Base mittels einer Säure oder eines stark sauren Ionenaustauschers abgespalten wird.

8. Verfahren nach Anspruch 1 bis 6, dadurch gekennzeichnet, daß der in der Position $Y_1$ stehende Säurerest mit Alkali abgespalten wird.

9. Mittel, bestehend aus oder enthaltend eine Verbindung der Formel I gemäß Anspruch 1.

10. Verwendung der Verbindungen der Formel I gemäß Anspruch 1 zur Behandlung des Bluthochdrucks.

11. Verwendung der Verbindungen der Formel I gemäß Anspruch 1 in Kombination mit einem Diuretikum zur Behandlung des Bluthochdrucks.

0052870

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 81109798.9

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.3) |
|---|---|---|---|
| | DE - A1 - 3 004 370 (MORTON-NORWICH) (14-08-1980)<br>* Patentansprüche; Seite 5, Zeilen 11-15; Beispiel 4 *<br>-- | 1,7,10 | C 07 D 209/42<br>C 07 D 215/48<br>C 07 D 217/26<br>C 07 D 401/12<br>A 61 K 31/40<br>A 61 K 31/47 |
| P | DE - A1 - 3 018 545 (MORTON-NORWICH) (27-11-1980)<br>* Seite 2, Zeilen 3-13; Beispiele 1,2 *<br>& BE-A1-883 320 (14-11-1980)<br>-- | 1,7,9,10 | |
| | EP - A1 - 0 012 845 (TANABE) (09-07-1980)<br>* Patentansprüche 1,4,6,8 *<br>-- | 1,7,10 | RECHERCHIERTE SACHGEBIETE (Int. Cl.3)<br>C 07 D 209/00<br>C 07 D 215/00<br>C 07 D 217/00<br>C 07 D 401/00<br>A 61 K 31/00 |
| | EP - A2 - 0 018 104 (TAKEDA) (29-10-1980)<br>* Patentansprüche 1,13,15 *<br>-- | 1,7,10 | |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-10
Unvollständig recherchierte Patentansprüche: –
Nicht recherchierte Patentansprüche: 11,12
Grund für die Beschränkung der Recherche:

Verfahren zur therapeutischen Behandlung des menschlichen oder tierischen Körpers (Art. 52(4) EPÜ)

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| WIEN | 15-02-1982 | ONDER |

0052870

Europäisches Patentamt

# EUROPÄISCHER TEILRECHERCHENBERICHT

Nummer der Anmeldung
EP 81109798.9

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| P | EP - A1 - 0 029 488 (HOECHST) (03-06-1981)<br>* Patentansprüche 1-4; Seite 7, Zeilen 18-22 * | 1,7-10 | |
| P | & DE-A1-2 937 779 (09-04-1981) | | |
| P | & DE-A1-2 946 909 (04-06-1981)<br>---- | | |
| | | | RECHERCHIERTE SACHGEBIETE (Int. Cl.³) |

EPA Form 1505.3 06.78